Europäisches Patentamt

European Patent Office

Office européen des brevets

(19)

(11) **EP 1 459 731 A1**

## (12) EUROPEAN PATENT APPLICATION

(43) Date of publication:
**22.09.2004 Bulletin 2004/39**

(51) Int Cl.⁷: **A61K 7/09**

(21) Application number: **04251039.6**

(22) Date of filing: **25.02.2004**

(84) Designated Contracting States:
**AT BE BG CH CY CZ DE DK EE ES FI FR GB GR
HU IE IT LI LU MC NL PT RO SE SI SK TR**
Designated Extension States:
**AL HR LT LV MK**

(30) Priority: **25.02.2003 US 373616**

(71) Applicant: **L'OREAL
75008 Paris (FR)**

(72) Inventors:
• **Darkwa, Adu Gyamfi
Olympia Field, Illinois 60461 (US)**

• **Osei-Acquah, Eric
Lynwood, Illinois 60411 (US)**
• **Cooper, James
Chicago, Illinois 60620 (US)**

(74) Representative: **Bentham, Stephen
J.A. KEMP & CO.
14 South Square
Gray's Inn
London WC1R 5JJ (GB)**

(54) **Hair relaxer system with mineral oil activator**

(57)    The present invention relates to an activator for use in a two-component hair relaxer system. The activator includes an alkaline material such as calcium hydroxide mixed with mineral oil. Preferably, the activator exists as a suspension, emulsion or gel. Hair relaxer systems including a mineral oil containing activator and a cream base, hair relaxers made by mixing these two components, and methods of using such systems to straighten hair are also described.

**Description**

BACKGROUND OF THE INVENTION

A no-lye hair relaxer is one that does not contain sodium or potassium hydroxide. Instead, it typically employs such bases as guanidine hydroxide. Because guanidine hydroxide is not stable for long periods in aqueous solutions, it must be prepared fresh just prior to using. Guanidine hydroxide is generally prepared by mixing an inorganic alkaline earth metal hydroxide with an aqueous solution of a salt of guanidine, where the anion of the salt is capable of being precipitated by the cation of the alkaline earth metal hydroxide. In commercially available products of this type, the guanidine hydroxide is generally prepared using calcium hydroxide and guanidine carbonate.

Two-component systems for the preparation and use of guanidine hydroxide relaxers are well documented in the patent literature, as disclosed, for example, in U.S. Patent No. 4,304,244. Other patents describing two-component systems include U.S. Patents Nos. 5,565,216, 5,679,327 and 6,435,193. Most two-component hair relaxer systems comprise a first component, the cream base, which often includes a water-containing cream base having a water-soluble salt of a relatively strong base with an anion capable of being precipitated by an alkaline earth metal ion under highly alkaline conditions. The second component, also generally known as the activator, usually contains an alkaline material having an alkaline earth metal ion which forms a precipitate with the anion when the first component and second component are mixed. U.S. Patent No. 5,565,216 describes these alkaline materials as being calcium hydroxide, barium hydroxide and strontium hydroxide, or an alkaline earth metal oxide such as calcium oxide or barium oxide. These all form corresponding alkaline earth metal hydroxides when placed in an aqueous medium. Mixtures of alkaline earth metal hydroxides and alkaline earth metal oxides are also possible.

The activator is mixed with the cream base, for example, one containing guanidine carbonate, prior to use and insoluble calcium carbonate and soluble guanidine hydroxide are formed. This is the basic chemistry behind most two-component hair relaxer systems.

When the activator disclosed in U.S. Patent No. 5,565,216 is formulated in the form of a lotion, it is a viscous, pourable suspension containing an alkaline material such as an alkaline earth metal hydroxide. Calcium hydroxide is preferred and generally is provided in an amount ranging from as low as 20 to as much as 60 weight percent. However, most preferably, the amount used was 40 to 50 weight percent. The activator lotion also contains a suitable water-miscible hydrophilic liquid carrier to deliver the alkaline material into the aqueous, continuous phase of the cream base emulsion. Suitable carriers were identified as including polyhydroxy compounds (such as propylene glycol, glycerine, butylene glycol and hexylene glycol), and ethers including simple ethers and polyethers. A preferred carrier is propylene glycol. The propylene glycol also acts as a humectant in the hair to prevent dryness. The amount of carrier in the lotion is preferably from 40 to 80% by weight, more preferably 50% to 60%. Notwithstanding the advantages obtained by the use of such activators, there is always room for improvement.

First, in practice, activators are generally highly alkaline, often more than 40% alkaline material. This dramatically limits the types and amounts of other ingredients which can be used to those stable at such high pHs. Along with generally higher amounts of alkaline materials (alkaline earth metal hydroxides and oxides) also come issues of stability. The use of generally higher levels of, for example, calcium hydroxide, can also leave dull residue and typical formulations often do not rinse out very well. Certain vehicles employed in the production of conventional activators, such as polyhydroxy compounds, can interfere with the degree of straightening or relaxation. The use of lower amounts of calcium hydroxide would also be desirable as it would likely reduce the demand for water soluble salts in the cream base, such as guanidine carbonate, potassium citrate or ion exchange or chelating compositions such as those disclosed in U.S. Patent No. 6,435,193.

SUMMARY OF THE INVENTION

The present invention addresses a number of these issues by providing an activator for a two-component hair relaxer system which can be used almost universally with any type of water soluble salt containing cream base or bases containing chelates or ion exchange materials such as known in the art. The activator of the present invention is a mixture of an alkaline material, preferably an alkaline earth metal oxide or hydroxide, and most preferably calcium hydroxide, and an oil, which is mineral oil, vegetable oil or mixtures, preferably in the form of a suspension, gel, or emulsion of two or more phases. While the invention will often be described in terms of a gel, it will be understood that emulsions and suspensions are also contemplated. The use of mineral oil provides for a more stable activator, which yields superior results, even with the use of lower levels of alkaline materials. Mineral oil does not affect the degree of straightening, is relatively easy to rinse from the hair without leaving dull residue. Furthermore, the lower quantities of base can result in minimal ashing. The lower levels of alkaline materials used also permit the use of lower concentrations of water soluble salts in the cream base, which can improve comfort, safety and performance. The lower levels of these materials also aid in formulation. Because of the lower overall concentration of hydroxide, the pH of the formulation is less basic, permitting the use of other ingredients which might be particularly sensitive to highly basic environments. Most importantly, however, even with a lower level of active ingredients, the performance of hair relaxing systems including the activator of the present invention is equal to or superior to those previously known.

Thus, in one aspect, the present invention relates to an activator for a two-component hair relaxer system including an effective amount of at least one alkaline material dispersed in mineral oil. Preferably, the activator includes an additive which can include, *inter alia,* a solubilizer, emulsifier and/or gelling agent and the activator is preferably in the form of a suspension, emulsion or gel. Most preferably, the activator includes both a solubilizer and a gelling agent.

The present invention also contemplates kits containing the activator of the present invention, as well as a cream base and methods of straightening or relaxing hair using a composition produced by admixing a predetermined amount of the activator of the present invention and one or more cream bases. Thus, another aspect of the present invention is a two-component hair relaxer system including an activator having an effective amount of at least one alkaline material dispersed in a mineral oil as well as a cream base. Most preferably, the activator includes both a solubilizer and a gelling agent.

In one aspect of the present invention, there is provided an activator for a two-component hair relaxer system. The activator includes an effective amount of at least one alkaline material dispersed in a mineral oil, suspension, emulsion or gel. Preferably, the alkaline material is an alkaline earth metal oxide or hydroxide. While the activator can contain any amount of such alkaline material which is capable of being dispersed in a suspension, emulsion or gel of mineral oil, the amount of alkaline material can generally range from as little as about 10% to as much as about 75% by weight of the total formulation (assuming a dry, anhydrous alkaline material). More preferably, the alkaline material is present in an amount of about 40% or less and more preferably between about 20% and 40% by weight. In addition to solubilizers and gelling and emulsifying agents, the activator of the present invention may also include conditioners, pigments, viscosity modifiers, fragrances, coloring agents, stabilizers and preservatives and other excipients known in the personal care and cosmetic industries. Methods of producing this activator and methods of its use are also described.

Another aspect of the present invention relates to a two-component hair relaxer system. This hair relaxer system includes an activator as discussed above and a cream base containing at least one component capable of reacting with the at least one alkaline material contained in the activator. Such cream bases include, without limitation, traditional water soluble salts such as guanidine carbonate, citrates such as potassium citrate, as well as ion exchange or chelating compositions such as those disclosed in U.S. Patent No.6,435,193. Finally, the invention includes a relaxer which is a mixture of the mineral oil based activator of the invention and a cream base, and methods of using same by applying the resulting mixture to a subject's hair for a period of not more than 45 minutes, preferably not more than 30 minutes.

DETAILED DESCRIPTION

The alkaline materials useful in accordance with the present invention include Group II alkaline earth metal oxides or hydroxides. The alkaline earth metal oxides or hydroxides useful in accordance with the present invention include barium, strontium and calcium. The most preferred alkaline material is calcium hydroxide. The amount of alkaline material useful in accordance with the present invention can vary with the activator, the type of alkaline material used, the type and composition of the cream base, the form of the activator (gel or emulsion) and the like. Generally, as long as the desired amount of alkaline material can be dispersed or otherwise suspended, gelled or emulsified, it is contemplated. However, in general, the amount can range from between about 10 to about 75% by weight of the activator formulation. More preferably, the amount of such alkaline material can be less than about 40 weight percent and more preferably between about 20 and about 40 weight percent.

Mineral oil is typically the single largest component of the activators of the present invention. Mineral oil can be present in as little as about 20% and as much as about 75% by weight of the activator. However, more preferably, mineral oil is present in an amount of between about 40 and about 65 % by weight, and even more preferably between about 45 and about 65% by weight. Mineral Oil is a liquid mixture of hydrocarbons obtained from petroleum. In the United States, Mineral Oil may be used as an active ingredient in OTC drug products. When used as an active drug ingredient, the established name is *Mineral Oil*. The labeling name in the EU will be, Paraffinum Liquidum, when regulations for ingredient labeling under the 6th Amendment to the EC Cosmetics Directive go into effect. It is known by CAS Nos. 8012-95-1 and 8042-47-5 and is available in numerous grades and under numerous technical names including heavy mineral oil, light mineral oil, liquid paraffin, liquid petrolatum and paraffin oil. A non-comprehensive list of mineral oils and suppliers can be found in the International Cosmetic Ingredient Dictionary and Handbook, Seventh Edition, 1997, Edited by J. Wenninger and G.N. McEwen, Jr. Any type or grade of mineral oil may be used in accordance with the present invention. Blends of various grades and viscosities of mineral oils are also contemplated. Mineral oil could be replaced or blended with any vegetable oil such as olive oil, canola oil, or avocado oil. In addition, some orall of the oil could be replaced with synthetic hydrocarbon bases such as isopropyl palmitate, hydrogenated polyisobutane, isohexadecane, isododecane, and the like. Mineral oil with up to about 20% by weight of one or more of these is preferred. Mineral oil alone is most preferred. Particularly preferred mineral oils have a viscosity of about 70 saybolt and are available under the trade names CARNATION from Witco Corporation and DRAKEOL® 7 available from Penreco, 4401 Park Avenue, Dickinson, TX 77539-6933, having a viscosity (ASTM D 445) of 65/75 SUS@100°F and 10.8/13.6 CST@40°C. Some of the mineral oil content can come from additives such as VERSAGEL 750 M. However, preferably, the formulation will include mineral oil, *per se,* an amount of mineral oil added to the formulation as mineral oil, not as a part of some other commercial additive.

In addition to alkaline material and mineral oil, the activator in accordance with the presence invention generally includes solubilizers or emulsifiers and/or gelling agents. Often, the solubilizer can act as an emulsifier in the right system. Of course, as these systems are preferably anhydrous, emulsions are multiple phase systems where none of the phases are aqueous.

Preferred solubilizers and emulsifiers include surfactants and most preferably polyoxyethylene and polyoxypropylene ethers such as laureth, oleth, ceteh, etc. of 10 moles of ethylene oxide or less. Solubilizers are preferably present in an amount of between about 0.01 to about 10% by weight of the total activator formulation, more preferably the amount of solubilizer present is between 0.10 and about 5%, and more preferably between 0.50 and about 2.0%. Oleth-5 available from Croda, Inc., 7 Century Drive, Parsippany NJ is most preferred.

As gelling agents, any material can be used which can produce a gel with mineral oil and the alkaline material of the present invention. Any gelling agent for hydrocarbons may be used. These include silica and a number of products sold under the trademark VERSAGEL™, available from Penreco, 4401 Park Avenue, Dickinson, TX 77539-6933. VERSAGEL 750 M is a preferred gellant, which includes about 85% by weight of mineral oil, with the balance being hydrogenated butylene-ethylene-styrene copolymers and hydrogenated ethylene/propylene/styrene copolymers. VERSAGEL M 750 has a viscosity of 75,000 cPs@25°C, and a specific gravity of 0.82@25°C. Other VERSAGEL M series products, including M 200, M 500 and M 1600, can also be used. However, particularly high viscosity mineral oil containing gelling agents such as VERSAGEL 1600 generally require the use of a solubilizer such as Oleth-5. Indeed, Penreco sells a number of gelling agents under the VERSAGEL trade name, many of which can be used. These include: VERSAGEL ME (hydrogenated polyisobutene base); VERSAGEL MP (isopropyl palmitate base); VERSAGEL MC (isohexadecane base); and VERSAGEL MD (isododecane base).

Where present, gelling agents are provided in an amount generally from between about 10 to about 90% by weight of the formulation. The relatively higher amounts are generally indicated where the VERSAGEL will be a source of the mineral oil. More preferably, the amount of gellant will range from between about 20 to about 55% more preferably between about 20 and about 35% by weight of the total formulation.

Additionally, the activator may contain a desiccant in order to remove any water that may be present in the activator and to maintain the activator substantially anhydrous until the time of its use. Commercially obtained solid calcium hydroxide powder may sometimes contain small amounts of water, as shown by an off-white color, and the addition of a desiccant, insures that it will be substantially free of water. Suitable desiccants include simple anhydrides, polymeric anhydrides, or molecular sieves. A preferred desiccant is calcium oxide because it becomes calcium hydroxide when mixed with water.

The desiccant is generally present in amounts of from 0 to 20% by weight, preferably 1% to 15%, more preferably 3 to 10%. When using calcium oxide as a desiccant, the amount thereof is included in the determination of the amount of alkaline material present in the activator.

The activator may also contain a coloring agent or opacifying agent. Preferred coloring agents are pigments and dyes. Suitable pigments include metal oxide whiteners. A preferred whitener is titanium dioxide. The whitener is preferably present in the activator in an amount of from 0.5 to 5% by weight.

In addition, the activator may contain a thickener. Suitable thickeners include organic polymers such as hydroxypropyl cellulose and hydroxyethylcellulose, anhydrous aluminum silicate and hydrated magnesium aluminum silicate and colloidal clays. A preferred thickener is fumed silica. The thickener is preferably present in the activator in amounts of from 1 to 6% by weight, more preferably, 2% to 5% by weight. These formulations may also include conditioners, viscosity modifiers, fragrances, stabilizers and preservatives and other excipients known in the personal care and cosmetic industries.

In a preferred embodiment of the present invention, the activator is in the form of an emulsion or gel that is substantially free of water. This form is preferred from the standpoint of ease of mixing because the activator has a closer rheology to the cream base.

Any cream base that can be activated by the use of this activator may be used in accordance with the present invention. These include those described in, for example, U.S. Patent No. 5,565,216, the text of column 6, line 49 through column 9, line 5, is hereby incorporated by reference. In general, this form of cream base is present in the form of an oil-in-water emulsion and contains a water soluble salt of a relatively strong base with an anion capable of being precipitated by an alkaline material, and in particular, an alkaline earth metal ion under highly alkaline conditions. The water soluble salt is present in the aqueous phase of the oil-in-water emulsion. The relatively strong base that forms the water soluble salt is a base that provides a pH of preferably between 2 and 14 when in an aqueous medium. The relatively strong base generally is an organic nitrogen, and preferably is guanidine. Other organic bases which may be used in place of guanidine include N-methyl guanidine, dimethylaminoguanidine, acetamidine, dimethylaminoamidine, aminoamidine and acetamide.

The anion capable of being precipitated by an alkaline material under highly alkaline conditions preferably is the carbonate ion. Thus, the preferred water-soluble salt is guanidine carbonate. Salts other than the carbonate salt, such as a sulfate, sulfite, phosphate, fluoride, oxalate, tartrate, laurate or alginate salt can be used.

The oil phase generally contains anhydrous, lipophilic ingredients, which include an oleaginous material and an emulsifier.

Any suitable oleaginous material can comprise the oil phase for the cream base emulsion. Suitable oleaginous materials include petrolatum, mineral oil and mineral jellies, but also can include vegetable and animal derived oils and fats, and like unctuous emulsifiable materials. Preferred oleaginous materials are mineral oil and petrolatum or a mixture of the two. Particularly preferred is a petrolatum-mineral oil mixture, where the petrolatum and mineral oil are present in approximately equal weight amounts. At least 50 weight percent, preferably about 55 to about 75 weight percent of the oil phase is comprised of the oleaginous material such as the mineral oil-petrolatum mixture.

The oil phase may further comprise emulsifiers, present in an amount of about 5 to about 18 weight percent of the cream base. Suitable emulsifiers include nonionic emulsifiers, anionic emulsifiers, cationic emulsifiers, and amphoteric emulsifiers.

The oil phase comprises about 15 to about 55 weight percent, preferably about 25 to about 45 weight percent of the cream base. The oil phase of the cream base is emulsified with the aqueous phase of the cream base which is described as follows.

The aqueous phase of the cream base comprises water and the water-soluble salt of a relatively strong base (e.g., guanidine carbonate) which can be added in suitable amounts.

The concentration of water-soluble salt (e.g., guanidine carbonate) in the cream base is generally from 1 to 20% by weight, preferably from 2 to 15% by weight. More preferred concentrations of the water-soluble salt (e.g., guanidine carbonate) are from 1 to 10% by weight and most preferred is 6 to 8% by weight of the cream base.

The water may be present in the aqueous phase in amounts of 80 to 90 weight % and in the cream base in amounts of 40 to 60 weight %. Water preferably comprises at least 50 weight percent of the cream base.

One or more co-emulsifiers may be added to the aqueous phase of the cream base to improve the texture of the cream base and control viscosity. A particularly preferred co-emulsifier is PPG-12 PEG-65-Lanolin oil which is a polyoxypropylene, polyoxyethylene derivative of lanolin oil, such as sold under the tradename Fluilan AWS by Croda, Inc., New York, N.Y. Lanolin derivatives may be added in an amount of about 1 to about 3 weight percent of the cream base.

A conditioner may be added to the cream base as desired. Suitable conditioning agents include non-polymeric quaternary nitrogen containing compounds such as those taught in U.S. Patent No. 5,077,042. Particularly preferred non-polymeric quaternary nitrogen containing conditioners include Finquat CT (Quaternium 75 sold by Fintex, Inc., Elmwood Park, N.J.) and Arquad 2HT-75 (Quaternium 18 manufactured by Akzo Chemical, Inc., Chicago, Ill.). Amounts of conditioner are preferably about 0.05 to about 5 weight percent, more preferably about 0.1 to about 4 weight percent of the cream base. Although polymeric quaternary nitrogen containing conditioners can also be used, they are not preferred for use in a guanidine carbonate-containing cream base of the present invention since they usually result in an unstable emulsion which separates upon aging and which must be remixed before use.

A surfactant may be added to the cream base as needed to enhance the rinsing of the relaxer from hair. The surfactant can comprise anionic or amphoteric compounds such as dioctyl sodium sulfosuccinate, lauroyl sarcosine, and cocoamphopropyl sulfonate.

Preferred surfactants for use with guanidine carbonate include Crodafos SG, Miranol C2M-SF, Miranol C2M, Sandopan DTC, and Duponol XL. Crodafos SG is PPG-5 ceteth-10 phosphate sold by Croda, Inc., New York, N.Y. Miranol C2M-SF is disodium cocoamphodipropionate sold by Miranol, Inc., Dayton, N.J. Miranol C2M is disodium cocoamphodiace-tate, also sold by Miranol. Sandopan DTC is sodium trideceth-7 carboxylate sold by Sandoz Chemical Corporation, Charlotte, N.C. Duponol XL is a surfactant containing DEA-lauryl sulfate, sodium lauraminopropionate and DEA-lauraminoproprionate, sold by DuPont Company, Wilmington, Del.

The surfactant may be present in the cream base in an amount of from 0.5% to 4%, preferably 1% to 3%, more preferably 1% to 2%, by weight of the cream base.

The cream base can also include cosmetic adjuvants, such as auxiliary emollients, auxiliary thickening agents, perfumes, preservatives, and product colorants.

The total amount of non-water components in the cream base preferably makes up no more than about 50% by weight of the total weight of the cream base.

The ratio of alkaline material containing activator (such as alkaline earth metal hydroxide containing activator) to cream base ranges generally from about 1 part by weight activator to about 4 parts by weight of cream base, to about 1 part by weight activator to about 10 parts by weight of cream base. Most preferably, 1 part by weight activator to about 6 parts by weight of cream base, to about 1 part by weight activator to about 8 parts by weight cream base.

Preferably a kit for a conditioning hair relaxer system embodying the principles of this invention comprises at least two packages. For example, a first package can include the cream base component (e.g., the guanidine carbonate containing cream base) as described above. A second package can include the activator component (e.g., the calcium hydroxide containing mineral oil gel activator). The contents of the first and second packages are admixed to provide an active relaxer composition just prior to use. In order to further minimize measuring errors, the activator component can be packaged in two separate packages, with each package containing one half of the total activator component. Then, when a consumer wishes to use the full contents of the kit, the consumer will empty the contents of both packages containing the activator composition into the contents of the first package. When the consumer wishes to use the half content of the kit, the consumer will measure out half the content of cream base package in a conventional manner and then empty one of the packages containing the activator into the measured half content of the cream base package. Since a single activator package contains exactly one half of the full amount of activator composition, no measuring error with respect to the activator is possible. The activator can be formulated to contain a 100% or greater molar excess of alkaline material, such as a 110% molar excess. If the activator component is then packaged in two separate packages, each package will contain the necessary number of moles to react with the full content of the cream base package. Thus, even if a consumer mistakenly uses a single package of activator with the full contents of the cream base package, there will be sufficient alkaline material to react with all of the water-soluble salt in the cream base.

The time of treatment of hair to be relaxed with the relaxer formulation of the present invention will normally be within the range of 5 to 45 minutes, starting from the first application of the relaxer composition to the hair. Generally, this treatment time will be at least 10 minutes, and there is normally no real upper limit on the time that the composition can remain on the hair. It is preferred to treat the hair for no more than about 30 minutes, preferably less than 25 minutes, and more preferably around 20 minutes.

After the above treatment time has elapsed, the relaxer composition should be removed from the hair in order to prevent damage to the treated hair. A major portion of the relaxer composition can be removed from the hair by thorough rinsing. It is preferred that the rinsing be followed by a neutralizing step, using any suitable agent that will neutralize alkali.

Further details of how to use a relaxer are disclosed in U.S. Patent No. 4,373,540, which patent is hereby incorporated by reference.

As previously noted, any type of cream base that would utilize an alkaline material containing activator as part of a two-component hair relaxer system may be used. These include the use of very different chemistries such as the use of ion exchange compounds and/or chelating agents as described in, *inter alia,* U.S. Patent No. 6,435,193 to Cannell *et al.,* issued August 20, 2003, the text of which pertaining to the formulation and use of analogous cream base compounds are hereby incorporated by reference. These include the disclosure at column 3, line 25 through column 5, line 5, column 5, line 14 through column 6, line 51, column 7, lines 12-19, and the examples which explain the production of such cream bases.

The complexing agent and the alkaline material may form a complex that, in most cases, has stronger interactions between the at least one complexing agent and the multivalent metal ion than the interactions between the multivalent metal and the hydroxide ion. As a result, the at least one complexing agent effectively removes the multivalent metal from the reaction medium and allows the equilibrium to be shifted.

Thus, the at least one cation exchange composition, also referred to as a chelating agent, can be used in combination with at least one complexing agent to modulate or control the rate of release of hydroxide ions from the at least one multivalent metal hydroxide, such as $Ca(OH)_2$, thereby producing a mixed composition for gentler relaxing and/or partial relaxing. A mixture of at least one complexing agent and at least one cation exchange composition may increase relaxing efficiency.

In a multicomponent kit, for example, the at least one cation exchange composition may be formulated with the component comprising at least one multivalent metal hydroxide or with the component comprising at least one complexing agent or itself may be a third component that is combined with one or both of the component comprising at least one multivalent metal hydroxide and the component comprising at least one complexing agent.

The at least one complexing agent of the present invention includes, but is not limited to, chelating agents and sequestering agents. A chelating agent is a compound or ligand that can bind to a metal ion, usually through more than one ligand atom, to form a chelate. See Lewis, R. J., Hawley's Condensed Chemical Dictionary p. 240 (1997). A chelate is usually a type of coordination compound in which a central metal ion, such as $Co^{2+}$, $Ni^{2+}$, $Cu^{2+}$, $Ca^{2+}$ or $Zn^{2+}$, is attached by coordinate links to two or more nonmetal atoms, e.g., ligands, in the same molecule. Non-limiting examples of common chelating agents include ethylenediaminetetraacetic acid (EDTA), nitrilotriacetic acid, and ethylenegylcol-bis(β-amino-ethyl ether)-N, N-tetraacetic acid.

Sequestering agents may be any material that prevents at least one ion from exhibiting its usual properties due to close combination with that material. Id. at 991. Certain phosphates, for example, form a coordination complex with metal ions in solution so that the usual precipitation reactions may be prevented. For example, calcium soap precipitates are not produced from hard water treated with certain phosphates or metaphosphates. Id. Other non-limiting examples of sequestering agents include hydroxy carboxylic acids, such as gluconic acid, citric acid and tartaric acid.

As previously mentioned, the at least one complexing agent can be chosen from chelating agents and sequestering agents. Non-limiting examples of chelating agents and sequestering agents include amino acids and crown ethers. In one embodiment, the at least one complexing agent is chosen from amino acids, such as monosodium glutamate, which is a known calcium chelator.

The at least one complexing agent may also be chosen from phosphates demonstrating chelating and/or sequestering properties and silicates demonstrating chelating and/or sequestering properties. Non-limiting examples of phosphates demonstrating chelating and/or sequestering properties include tripotassium phosphate, and trisodium phosphate. Non-limiting examples of silicates demonstrating chelating and/or sequestering properties include disodium silicate and or dipotassium silicate.

Other non-limiting examples of the at least one complexing agent that may be useful in the practice of the invention include organic acids and salts thereof. The cations that may be used to form the salts of organic acids of the present invention may be chosen from organic cations and inorganic cations. In one embodiment, the inorganic cations are chosen from potassium, sodium and lithium.

In another embodiment, the at least one complexing agent is chosen from mono-hydroxycarboxylic acids, dihydroxycarboxylic acids, polyhydroxycarboxylic acids, mono-aminocarboxylic acids, di-aminocarboxylic acids, poly-aminocarboxylic acids, mono-hydroxysulfonic acids, di-hydroxysulfonic acids, polyhydroxysulfonic acids, mono-hydroxyphosphonic acids, dihydroxyphosphonic acids, polyhydroxyphosphonic acids, mono-aminophosphonic acids, diaminophosphonic acids and polyaminophosphonic acids.

In a further embodiment, the at least one complexing agent is chosen from ethylene diamine tetraacetic acid (EDTA), -(hydroxyethyl) ethylene diamine triacetic acid, aminotrimethylene phosphonic acid, diethylenetriamine-pentaacetatic acid, lauroyl ethylene diamine triacetic acid, nitrilotriacetic acid, iminodisuccinic acid, tartaric acid, citric acid, N-2-hydroxyethyliminodiacetic acid and salts of any of the foregoing.

In a further embodiment, the at least one complexing agent is chosen from a salt of EDTA, such as sodium EDTA, lithium EDTA, potassium EDTA and guanidine EDTA. EDTA has a strong calcium binding constant over a wide range of pH. For example, tetrasodium EDTA generally solubilizes calcium hydroxide in aqueous media to give a clear solution. The use of at least one complexing agent, such as tetrasodium EDTA, that solubilizes the multivalent metal ion of the at least one multivalent metal hydroxide may offer the benefit of no "ashing." However, the use of one or more complexing agents that do not completely solubilize the multivalent metal ion but only form slightly-soluble or sparingly-soluble complexing agent-multivalent metal ion complexes is also within the practice of the invention. Citrates are preferably used in an amount of about 5% to about 15% by weight based on the weight of the cream base, more preferably 5% to 10% by weight. These same ranges would apply to the use of ion exchange or chelating materials.

EXAMPLE 1

A cream base for a two-component hair relaxer system according to the present invention was formulated.

| Formulation of Relaxer Base | |
| --- | --- |
| | Weight Percent |
| Oil Phase | |
| Cosmowax J | 12.00 |
| Petrolatum | 10.00 |
| Light Mineral oil | 10.00 |
| PEG-5 Soya Sterol | 1.00 |
| (sold under the name Generol 122E-5 by Henkel Corp., Hoboken, N.J.) | |
| Water Phase | |
| Deionized Water | 54.7985 |
| PPG-12 PEG-65-Lanolin Oil | 3.00 |
| (sold under the name Fluilan AWS by Croda, Inc.) | |
| Guanidine Carbonate | 7.40 |
| Succinic Acid | 0.30 |

(continued)

| Formulation of Relaxer Base | |
| --- | --- |
| | Weight Percent |
| Water Phase | |
| Thiazole Yellow "G"<br>(sold by Pylam Products Co., Inc., Garden City, New York) | 0.0015 |
| PPG-5-Ceteth-10 Phosphate<br>(sold under the name Crodafos SG by Croda Inc.) | 1.50 |
| | 100.00 |

The pH of the relaxer cream base is 10.1.

To prepare the cream base, the components of the oil phase are placed together in a heatable vessel, and are heated to between 75° and 80°C.

In a separate heatable vessel, the above components of the water phase are added, and homogenized. The Fluilan AWS should be heated to 60° to 65°C. before it is added to the heatable vessel. The guanidine carbonate is thoroughly dissolved in the water phase, and then succinic acid is added to the water phase.

The vessel containing the water phase is then heated quickly to 70°C., and the heated oil phase is then added slowly to the water phase with moderate homogenization. The resulting emulsion is homogenized for 15 minutes more with moderate agitation. The homogenized emulsion is then transferred to a cooling vessel where it is cooled with stirring to 50°C. At 50°C., the Crodafos SG can be added, and mixing and cooling continue. The Thiazole Yellow G (a color indicator) can be added when the temperature reaches 45°C., and mixing is continued. 30 grams of activator would be used to activate 235 grams of cream base.

EXAMPLE 2

A relaxer base for a two-component hair relaxer system according to the present invention was formulated. The components had the following composition.

| Formulation of Relaxer Base Containing Guanidine Carbonate. | |
| --- | --- |
| | Weight % |
| Oil Phase | |
| Cetyl alcohol | 4.80 |
| Stearyl alcohol | 5.20 |
| Steareth-20 | 1.00 |
| Mineral oil | 10.00 |
| Petrolatum | 10.00 |
| Steareth-10 | 1.00 |
| Water Phase | |
| Deionized Water | 59.01 |
| Fluilan AWS | 3.00 |
| Disodium EDTA | 0.20 |
| Guanidine Carbonate | 5.59 |
| Succinic Acid | 0.2 |

The pH of the relaxer base is 10.28.

The relaxer base was prepared in a manner similar to that described in Example 1.

30 grams of activator would be used to activate 235 grams of cream base.

EXAMPLE 3

Hair Relaxing Cream Base Comprising a Complexing Agent

A cream base for a two-component hair relaxing composition was prepared. The cream base for a containing the complexing agent tetrasodium EDTA was prepared as follows:

| Materials | % w/w |
|---|---|
| Cetyl alcohol | 1.0 |
| Steareth-2 | 0.5 |
| Steareth-10 | 2.5 |
| Mineral Oil | 15.0 |
| Petrolatum | 5.5 |
| Cetearyl alcohol and Cetearyl Phosphate | 7.5 |
| Propylene Glycol | 3.0 |
| Tetrasodium EDTA | 30.5 |
| Water | 34.5 |

The hair relaxer cream base in accordance with this example can be formulated using the procedure generally described in connection with Example 1. 30 grams of activator would be used to activate 235 grams of cream base.

EXAMPLE 4

A potassium carbonate containing cream base useful in accordance with the invention include those having the following formula:

| INCI Name Ingredients | Trade Name | % w/w |
|---|---|---|
| Petrolatum | Snow White Petrolatum | 16.50% |
| Mineral Oil | Carnation Oil | 13.50% |
| Cetearyl Alcohol | TA 1618 F | 4.80% |
| Behentrimonium Methosulfate | Incroquat Behenyl TMS | 4.00% |
| PEG-75 Lanolin | Super Solan LD2845 | 1.20% |
| Water | Deionized Water | 48.90% |
| Cocamidopropyl Betaine | Lexaine C | 1.25% |
| Propylene Glycol | | 1.00% |
| Potassium Carbonate | | 3.85% |
| Water | Deionized Water | 5.00% |
| Polyquaternium-6 | Merquat 100 | 0.00% |
| TOTAL | | **100.0000%** |

Into kettle equipped with double-action mixer (Lightnin' and sweep mixer) add petrolatum, mineral oil, cetearyl alcohol, behentrimonium methosulfate and PEG-75 lanolin. Begin heating to 80°C. (Start mixing slowly when waxes melt sufficiently). Charge water (46.15%) into separate kettle equipped with Lightnin mixer. Add cocamidopropyl betaine and propylene glycol. Heat and mix kettle to 85°C. When both oil and water phases are at maximum temperature, slowly add water phase to oil phase using Lightnin' mixer at moderate agitation. Once water phase addition is completed, mix batch for 30 minutes. Slowly cool to 35°C, switching to sweep mixer when batch thickens. Slowly add premix solution of [water (5%) and potassium carbonate] mixing well. Continue cooling and mixing further to 22-25°C, versate batch and then sample. Batch was smooth and stable. 230 grams of this cream base can be activated by 30 grams of the activator of Example 7.

EXAMPLE 5

A potassium carbonate containing cream base useful in accordance with the invention include those having the following formula:

| INCI Name Ingredients | Trade Name | % w/w |
|---|---|---|
| Petrolatum | Snow White Petrolatum | 16.50% |
| Mineral Oil | Carnation Oil | 13.50% |
| Cetearyl Alcohol | TA 1618 F | 7.80% |
| PEG-75 Lanolin | Super Solan LD2845 | 1.20% |
| Water | Deionized Water | 43.80% |
| Cocamidopropyl Betaine | Lexaine C | 1.25% |
| Propylene Glycol | | 1.00% |
| Potassium Carbonate | | 3.45% |
| Water | Deionized Water | 5.00% |
| Hexadimethrine Chloride | Mexomere PO | 2.00% |
| Water | Deionized Water | 1.50% |
| Polyquaternium-6 | Merquat 100 | 1.50% |
| Water | Deionized Water | 1.50% |
| TOTAL | | 100.0000% |

Into kettle equipped with double-action mixer (Lightnin' and sweep mixer) add petrolatum, mineral oil, cetearyl alcohol, behentrimonium methosulfate and PEG-75 lanolin. Begin heating to 80°C. (Start mixing slowly when waxes melt sufficiently). Charge water (43.80%) into separate kettle equipped with Lightnin' mixer. Add cocamidopropyl betaine and propylene glycol. Heat and mix kettle to 85°C. When both oil and water phases are at maximum temperature, slowly add water phase to oil phase using Lightnin' mixer at moderate agitation. Once water phase addition is completed, mix batch for 30 minutes. Slowly cool to 35°C, switching to sweep mixer when batch thickens. Slowly add premix solution of [water (5%) and potassium carbonate]. Mix well, then add premix solutions [hexadimethrine chloride and water (1.5%)] and [water (1.5%) and polyquaternium-6] mixing well between each solution addition. Continue cooling and mixing. Continue mixing and cool batch further to 22-25°C, versate batch and then sample. Product remained lotion like even at 32°C. Addition of hexadimethrine chloride and polyquaternium-6 had no positive nor negative effect - product still lotion-like at 25°C. behentrimonium methosulfate has a stabilizing effect. Oven sample separated after 24 hrs -- clear layer on bottom of jar. Room temperature sample still had lotion-like viscosity. 230 grams of this cream base can be activated by 30 grams of the activator of Example 7.

EXAMPLE 6

Citrate containing cream base useful in accordance with the invention include those having the following formula:

| INCI Name Ingredients | Trade Name | % w/w |
|---|---|---|
| Petrolatum | Snow White Petrolatum | 16.50% |
| Mineral Oil | Carnation Oil | 13.50% |
| Cetearyl Alcohol | TA 1618 F | 4.50% |
| Behentrimonium Methosulfate | Incroquat Behenyl TMS | 5.50% |
| PEG-75 Lanolin | Super Solan LD2845 | 1.50% |
| Water | Deionized Water | 36.75% |
| Cocamidopropyl Betaine | Lexaine C | 1.25% |
| Propylene Glycol | | 1.00% |
| Potassium Citrate | | 9.50% |
| Water | Deionized Water | 10.00% |
| Polyquatemium-6 | Merquat 100 | 0.00% |
| TOTAL | | 100.0000% |

Into kettle equipped with Lightnin' and sweep mixer add petrolatum, mineral oil, cetearyl alcohol, behentrimonium methosulfate and PEG-75 lanolin. Begin heating to 80°C. (Start mixing slowly when waxes melt sufficiently). Charge water (36.75%) into separate kettle equipped with Lightnin mixer. Add cocamidopropyl betaine and propylene glycol. Heat and mix kettle to 85°C. When both oil and water phases are at maximum temperature, slowly add water phase to oil phase using Lightnin' mixer at moderate agitation. Once water phase addition is completed, mix batch for 30 minutes. Slowly cool to 30°C, switching to sweep mixer when batch thickens. Slowly add premix solution of [water (10%) and hexadimethrine chloride] mixing well. Continue mixing and cool batch further to 22- 25°C, versate batch and then sample. Centrifuge shows 0.25 ml ofwater on bottom. Oven sample shows stability thus far. 230 grams of this cream base can be activated by 30 grams of the activator of Example 7.

EXAMPLE 7

A gelled mineral oil activator in accordance with the present invention was produced using the following ingredients.

| INCI Name Ingredients | Trade Name | activity | % w/w |
|---|---|---|---|
| Mineral Oil | Drakeol #7 | 100% | 38.50% |
| Oleth-5 | Eumulgin O5 S | 100% | 1.00% |
| Mineral Oil (and) Hydrogenated Butylene/Ethylene/Styrene Copolymer (and ) Hydrogenated Ethylene/Propylene/Styrene Copolymer | Versagel 750 M | 100% | 25.00% |
| Calcium Hydroxide | Hydrated Lime | 100% | 35.00% |
| Titanium Dioxide | Titration | 100% | 0.50% |

Into kettle equipped with Cowles mixing blade, add mineral oil and oleth-5. Mix well then add Versagel 750 M. Heat and mix to 90°C. Once uniform, switch to homogenizer and mix at highest allowable level without aerating batch. Slowly sift in calcium hydroxide mixing until well "wetted". Add titanium dioxide and continue mixing. Once batch is uniform, switch to side-sweep mixer and cool batch to 25°C. Stop processing and sample batch.

| Specifications: | | |
|---|---|---|
| Test | Apparatus | Specifications |
| Color | Visual | Off-white; To match standard |
| Odor | Olfactory | To match standard |
| Appearance | Visual | Thick opaque lotion; To match standard |
| pH (@25°C) | PH Meter | Not performed |
| Viscosity (Initial @25°C) | Rheomat 180; Spindle 3; 1-min. | 60$\pm$5 DU |
| Viscosity (24-hrs; @25°C) | Rheomat 180; Spindle 3; 1-min. | 70$\pm$5 DU |
| Relative Density (@25°C) | Pycnometer | 1.09$\pm$0.01 |
| Calcium Hydroxide (%) | Titration | 35$\pm$1.75% |

EXAMPLE 8

A cream base useful for a relaxer system including the mineral oil activator of the present invention can be produced as follows:

| A | Petrolatum | 16.5 |
|---|---|---|
| | Mineral Oil | 13.5 |
| | Cetearyl Alcohol Behentrimonium Methosulfate, PEG-75 Lanolin | 10 |
| B | Propylene Glycol | 1 |
| | Cocamidopropyl Betaine | 1.25 |
| | Polyquaternium-6 | 1.5 |
| C | Water | 50.336 |
| | Guanidine Carbonate | 5.7 |
| | Succinic Acid | 0.001 |
| D | Fragrance | 0.2 |
| | Orange 4 CI 15510 | 0.003 |
| E | Premix Bittering Agent * | 0.01 |
| | Total | 100 |

*

| Water | 96 |
|---|---|
| Denatonium | 4 |
| | |
| Total | 100 |

Preparation of the solution in the homomixer kettle

Procedure Part A:

1. Charge into the kettle the whole amount of (water) required for the batch (Part C) and start the Homomixer (moderate to high speed). Weigh exactly the (guanidine carbonate) amount and add and mix until all crystals are dissolved. A 6300 kg batch normally takes 45 minutes. Add the (succinic acid).

(Part A) Preparation at the sweep tank

2. Charge into the Sweep Tank (Part A) ((petrolatum), (mineral oil) and (cetearyl alcohol/ Behentrimonium Methosulfate/ PEG-75 Lanolin mixture)). Melt all components by heating to 75°C - 80 °C. When 75 °C - 80°C is reached, stop steam.

Combining the (Part A) to the (guanidine carbonate) solution (Part C) in the homomixer kettle

3. Start heating the (guanidine carbonate) solution in Homomixer Kettle (part C) to 50 °C. At 50 °C, add (propylene glycol), (cocamidopropyl betaine) and (polyquaternium-6) (Part B) and start the mixer. The phase temperature for this phase is 50 °C (moderate to high speed).
4. Pump the (Part A) which is at 75°C - 80 °C at the Sweep Tank into the bottom of the Homomixer Kettle and homomix for not more than 10 minutes. After 10 minutes, transfer the batch back in the Sweep Tank with homogenizer on and pump at 36 Hz. Air foam will be generated at this point. Take all necessary steps to deaerate the batch before any cooling starts. Recirculate batch for 2-5 minutes to clear oil from transfer line.

Sweep Tank

5. When the batch is fully transferred, let it deaerate by slow to moderate sweep agitation. Temperature is about 65°C - 70°C.

6. Premix (Part D) at the blending Area while cooling the batch to 55°C - 60°C at the Sweep Tank. Use city water-cooling at this point.

7. At 55 °C - 60 °C, add (Part D) premix to the batch. Begin chill water-cooling at 35°C - 50°C with sweep mixing. As it thickens, increase the mixing accordingly. Recirculation can be started at this point.

8. At 30°C, add the (premix bittering agent) and continue mixing and recirculation for about 30 minutes.

EXAMPLE 9

A cream base useful for a relaxer system including the mineral oil activator of the present invention can be produced as follows:

| | | |
|---|---|---|
| A | Petrolatum | 16.5 |
| | Mineral Oil | 13.5 |
| | Cetearyl Alcohol Behentrimonium Methosulfate, PEG-75 Lanolin | 10 |
| | | |
| B | Propylene Glycol | 1 |
| | Cocamidopropyl Betaine | 1.25 |
| | Polyquaternium-6 | 1.5 |
| | | |
| C | Water | 49.836 |
| | Guanidine Carbonate | 6.2 |
| | Succinic Acid | 0.001 |
| | | |
| D | Fragrance | 0.2 |
| | Orange 4 CI 15510 | 0.003 |
| | | |
| E | Premix Bittering Agent * | 0.01 |
| | | |
| | Total | 100 |

| | | |
|---|---|---|
| * | Water | 96 |
| | Denatonium | 4 |
| | | |
| | Total | 100 |

This cream base may be produced using the procedures described in Example 8.

EXAMPLE 10

The activator of the present invention was compared to conventional activators such as those described in U.S. Patent No. 5,565,216. To demonstrate the properties of conventional products, two SoftSheen Carson hair relaxer products sold under the trademark Dark and Lovely were used. These two products were regular strength and super strength. The cream base for the regular strength product included approximately 6.5% guanidine carbonate while the super strength had approximately 7% guanidine carbonate. The activator used in each case contained 45% calcium hydroxide and about 52% propylene glycol. These were compared with formulations in accordance with the present invention where the regular strength cream base contained 5.7% guanidine carbonate (Example 8) and the super strength cream base contained 6.2% guanidine carbonate (Example 9). The mineral oil activator of the present invention used was that described in Example 7.

Normal brown hair from one individual was relaxed with the relaxers produced by blending the above components. 117.5 grams of each cream base and 15 grams of each activator were mixed and applied for 20 minutes at room temperature. The relaxed hair was then rinsed and shampooed with Color Signal Shampoo. Normal brown hair was also included in the test as a control. The hair was stretched in water, with 70 hair fibers being used per test.

Effect of relaxers on wet tensile strength of dark brown hair

n=70 fibers

| Hair Type | Cross-section sq. micron | Load at 15%, gmf | Load at break, gmf |
|---|---|---|---|
| Normal brown hair | 6,570+/-1,530 | 33.7+/-6.2 | 113.5+/-21.9 |
| Regular strength prior art | 8,210+/-2,212 | 17.4+/-4.8 | 79.7+/-19.8 |
| Regular strength Invention | 7,490+/-1,916 | 16.1+/-3.3 | 76.6+/-15.5 |
| t-test | 0.056 | 0.08 | 0.211 |
| Super strength prior art | 8,770+/-2,292 | 13.6+/-3.1 | 68.4+/-15.1 |
| Super strength Invention | 7,910+/-1,565 | 15.3+/-2.9 | 78.5+/-14.7 |
| t-test | 0.0202 | 0.0035 | 0.000436 |

| Hair Type | Young's Modulus, Mpa | Work-15%, J/m2 | Post-Yield Modulus, MPa | Total work, J/m2 |
|---|---|---|---|---|
| Normal brown hair | 453+/-50 | 200+/-20 | 381+/-55 | 1,450+/-270 |
| Regular strength prior art | 111.0+/-63.0 | 77.2+/-15.0 | 229+/-38 | 838+/-186 |
| Regular strength Invention | 113.7+/-61.1 | 80.5+/-21.5 | 247+/-49 | 843+/-197 |
| t-test | 0.68 | 0.3065 | 0.022 | 0.8815 |
| Super strength prior art | 61.9+/-19.1 | 58.0+/-12.6 | 194+/-37 | 663+/-138 |
| Super strength Invention | 90.1+/-26.88 | 71.2+/-15.5 | 234+/-41 | 834+/-166 |
| t-test | 2.98E-09 | 1.47E-06 | 3.30E-08 | 1.58E-08 |

As shown in the cross-section measurements, relaxed hair, compared to the control, became larger in texture. The swelling is one of the indications of being more porous and more damaged. Hair relaxed with the formulation of the present invention swelled significantly less than the one relaxed with the prior art formula. This signified less damage.

As far as the tensile properties are concerned, the load at 15%, work at 15%, load at break, total work at break, Young's Modulus and post-yield modulus were all measured. Results from the relaxed hair using both regular formulations were mixed. Some of the properties from the prior art formulation were better than some of the properties of the formulation of the present invention and some were worse. These differences were not statistically significant, as shown by t-tests. Therefore, the tensile properties of hair relaxed with both formulations at "regular" strength were about the same. What is significant, however, is that the formulation of the present invention accomplished these comparable results with significantly less calcium hydroxide, 35% versus 45%. The cream base used also included less guanidine carbonate.

At the "super" strength, hair relaxed with the formulation of the present invention exhibited stronger tensile properties than the one relaxed with the prior art formulation. The results demonstrate that in all the properties measured, the present invention was significantly better than the prior art. Therefore, the tensile properties of hair relaxed with the present invention in a "super" strength are better than the art. It is noteworthy that in both the regular and super strength versions of the prior art formulation, the amount of guanidine carbonate was also higher in the cream base. Indeed, the "super" strength of the prior art formulation, having the highest guanidine carbonate and calcium hydroxide content provided the worst results overall.

**Claims**

1. An activator for a two-component hair relaxer system composing: an effective amount of at least one alkaline material dispersed in mineral oil.

2. The activator of claim 1, wherein said at least one alkaline material is an alkaline earth metal hydroxide or oxide.

3. The activator of claim 2, wherein said alkaline earth metal oxide or hydroxide is $Ca(OH)_2$.

4. The activator of claim 1, wherein said alkaline material is provided in an amount of between about 10% and about 75% by weight of said activator.

5. The activator of claim 4, wherein said alkaline material is provided in an amount of about 40% or less based on the weight of said activator.

6. The activator of claim 5, wherein said alkaline material is $Ca(OH)_2$ and is provided in an amount of between about 20% and about 40% by weight of said activator.

7. The activator of claim 1, wherein said mineral oil is provided in an amount of between about 20% and about 75% by weight of said activator.

8. The activator of claim 7, wherein said mineral oil is provided in an amount of between about 40% and about 65% by weight of said activator.

9. The activator of claim 8, wherein said mineral oil is provided in an amount of between about 45% and about 65% by weight of said activator.

10. The activator of claim 1, further comprising: an additive selected from solubilizers, emulsifiers and gelling agents.

11. The activator of claim 10, wherein said activator is in the form of a gel.

12. The activator of claim 10, wherein said activator is in the form of an emulsion.

13. The activator of claim 10, wherein said activator is in the form of a suspension.

14. The activator of claim 1, further comprising a solubilizer, said solubilizer being provided in an amount of between about 0.01% and about 10% by weight of the total activator formulation.

15. The activator of claim 1, further comprising a gelling agent, said gelling agent being provided in an amount of between about 10% and about 90% by weight of the total activator formulation.

16. The activator of claim 1, further comprising a second oil selected from the group consisting of vegetable oils and synthetic hydrocarbon bases.

17. An activator for a two-component hair relaxer system comprising between about 10% and about 75% by weight of $Ca(OH)_2$, between about 20% and about 75% by weight of mineral oil, between about 0.01% and about 10% by weight of a solubilizer and between about 10% and about 90% by weight of a gelling agent.

18. A two-component hair relaxer system comprising: an activator including an effective amount of at least one alkaline earth metal oxide or hydroxide dispersed in a mineral oil and a cream base.

19. The two-component hair relaxer system of claim 18, wherein said cream base includes a water soluble salt of a relatively strong base providing a pH of between 2 and 14 when in an aqueous medium an ion exchange compound or chelating agent or a citrate.

20. The two-component hair relaxer system of claim 19, wherein said cream base includes an ion exchange compound or chelating agent.

21. The two-component hair relaxer system of claim 19, wherein said cream base includes a citrate.

22. A hair relaxer comprising a mixture of an activator comprising an effective amount of at least one alkaline material dispersed in mineral oil which has been mixed with a cream base.

**European Patent Office**

## EUROPEAN SEARCH REPORT

Application Number

EP 04 25 1039

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | US 4 304 244 A (DE LA GUARDIA MARIO J) 8 December 1981 (1981-12-08) * claims 1-23; examples 1-16 * | 1-22 | A61K7/09 |
| P,X | US 2004/005284 A1 (CANNELL DAVID W ET AL) 8 January 2004 (2004-01-08) * paragraph [0035] * * paragraph [0037] - paragraph [0046] * * paragraph [0053] - paragraph [0074] * * claims 1-12,23,24,36,37 * | 1-22 | |
| X | WO 01/64171 A (OREAL ; CANNELL DAVID W (US); NGUYEN NGHI VAN (US)) 7 September 2001 (2001-09-07) * claims 15-33; examples 2,6,7 * | 1-22 | |
| X | US 4 605 018 A (DE LA GUARDIA MARIO ET AL) 12 August 1986 (1986-08-12) * examples 2-5 * | 1-22 | |

TECHNICAL FIELDS SEARCHED (Int.Cl.7)

A61K
A61Q

The present search report has been drawn up for all claims

| Place of search | Date of completion of the search | Examiner |
|---|---|---|
| Munich | 20 July 2004 | Szarek, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**                 EP 04 25 1039

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2004

| Patent document cited in search report | | Publication date | Patent family member(s) | | Publication date |
|---|---|---|---|---|---|
| US 4304244 | A | 08-12-1981 | FR | 2393572 A1 | 05-01-1979 |
| | | | GB | 1600807 A | 21-10-1981 |
| | | | KE | 3452 A | 05-10-1984 |
| | | | WO | 8001038 A1 | 29-05-1980 |
| | | | US | 4314572 A | 09-02-1982 |
| | | | US | 4324263 A | 13-04-1982 |
| | | | US | 4303085 A | 01-12-1981 |
| | | | US | 4373540 A | 15-02-1983 |
| | | | ZA | 7803133 A | 30-05-1979 |
| US 2004005284 | A1 | 08-01-2004 | NONE | | |
| WO 0164171 | A | 07-09-2001 | US | 6562327 B1 | 13-05-2003 |
| | | | AU | 4331801 A | 12-09-2001 |
| | | | BR | 0108907 A | 24-12-2002 |
| | | | CA | 2401009 A1 | 07-09-2001 |
| | | | CN | 1407883 T | 02-04-2003 |
| | | | EP | 1261312 A2 | 04-12-2002 |
| | | | JP | 2003524658 T | 19-08-2003 |
| | | | WO | 0164171 A2 | 07-09-2001 |
| | | | ZA | 200206840 A | 04-04-2003 |
| US 4605018 | A | 12-08-1986 | NONE | | |

EPO FORM P0459

For more details about this annex : see Official Journal of the European Patent Office, No. 12/82